# EUROPÄISCHE PATENTSCHRIFT

(11) **EP 0 361 229 B1**
(45) Veröffentlichungstag und Bekanntmachung des Hinweises auf die Patenterteilung: **27.04.1994**
(21) Anmeldenummer: 89117097.9
(22) Anmeldetag: 15.09.1989
(51) Int. Cl.: C09B 69/10, C12Q 1/68, G01N 33/58

(54) **Polymer-gebundene-Farbstoffe, Verfahren zu deren Herstellung und Verwendung**
Polymer-bonded dyes, process for their preparation and their use
Colorants liés à des polymères, leur procédé et leur utilisation

(30) Priorität: 28.09.1988 DE 3832830; 30.06.1989 DE 3921498
(43) Veröffentlichungstag der Anmeldung: 04.04.1990
(73) Patentinhaber: BAYER AG, 51368 Leverkusen (DE)
(72) Erfinder: Hugl, Herbert, Dr., D-5060 Bergisch Gladbach 2 (DE); Bömer, Bruno, Dr., D-5060 Bergisch Gladbach 2 (DE); Kölbl, Heinz, Dr., West Haven Connecticut 06516 (US); Seng, Florin, Dr., D-5060 Bergisch Gladbach 2 (DE); Kuckert, Eberhard, Dr.,c/o Molecular Diagn.Inc., West Haven,CT 06516 (US); Sackmann, Günter, Dr., D-5090 Leverkusen 3 (DE)

(56) Entgegenhaltungen:
- FR-A- 2 390 731
- US-A- 4 166 105

## Beschreibung

Die vorliegende Erfindung betrifft Polymer-gebundene-Farbstoffe, Verfahren zu deren Herstellung und deren Verwendung wie zum Beispiel als Markierungssubstanzen in Analysenverfahren. Die Polymer-gebundenen Farbstoffe, auch Polymere Farbstoffe genannt, enthalten verknüpfbare funktionelle Gruppen und sind unter üblichen Analysenbedingungen wasserlöslich. Verantwortlich für diese Wasserlöslichkeit ist normalerweise der Polymeranteil. Die Farbstoffe an sich sind oft wasserunlöslich.

Die Aufgabe die der vorliegenden Erfindung zugrunde lag, war es, neue Markierungssubstanzen zu entwickeln, welche in biologischen Testsystemen einsetzbar sind. Die Markierungssubstanzen sollen eine vergleichbare Nachweisempfindlichkeit aufweisen, wie die bekannten Markierungssubstanzen. Sie sollen aber nicht deren Nachteile haben, wie z.B. schlechte Arbeitssicherheit und nur verwendbar in Speziallabors (Radioaktivität) oder mangelhafte Stabilität (Enzymmarkierung).

Die erfindungsgemäßen polymeren Farbstoffe haben normalerweise mittlere Molekulargewichte in der Größenordnung von etwa M̅ₙ = 2 x 10³ bis etwa 5 x 10⁶ Dalton. Bevorzugt sind Molekulargewichte von etwa 10⁴ bis 10⁶ Dalton.

Unter üblichen Analysenbedingungen sind die Polymeren Farbstoffe in wäßrigen Medien zu mindestens 0,1 %, vorzugsweise mindestens zu 1 % löslich.

Unter üblichen Analysenbedingungen werden solche verstanden wie sie in biologischen Testen insbesondere in Bindungsanalysenverfahren wie z.B. Immunoassays oder Gensonden-Tests vorkommen. Zu nennen wären dabei beispielsweise Temperaturen von bis zu etwa 70° C, bevorzugt von ca. 10° C bis 40° C und pH-Werte von etwa 3 bis 11, bevorzugt von 5 bis 9. Bei besonderen Testen bzw. Analysenverfahren kann durchaus von diesen Werten abgewichen werden. Wesentlich ist die Wasserlöslichkeit der Polymer-gebundenen-Farbstoffe, weil dadurch der Einsatz der Farbstoffe in biologischen Analysenverfahren möglich ist.

Farbige Polymere und ihre Herstellung durch Umsetzung von Dicarbonsäureanhydridgruppen enthaltenden Polymeren mit vorzugsweise Aminogruppen enthaltenden Farbstoffen sind bekannt. So beschreiben Kalopissis und Viout in US-PS 3 915 635 Haarbehandlungsmittel, welche aus anhydridgruppenhaltigen Polymeren und Azo-, Anthrachinon-oder Benzolfarbstoffen mit Aminogruppen hergestellte polymere Farbstoffe enthalten. Die Polymeren sind jedoch nur in wäßrigen Alkoholen und nicht in Wasser löslich. Außerdem besitzen sie keine zusätzlichen Reaktivgruppen, die eine gezielte Verknüpfung mit z.B. Antikörpern oder DNA ermöglichen.

Hirschfeld beschreibt in US-PS 4 166 105 Reagenzien zum Nachweis spezifischer Reaktanten wie Antigene, die aus einem an einen Antikörper gebundenen Polymeren, welches eine Vielzahl von Farbstoffmolekülen enthält, bestehen.

Die Farbstoffpolymeren besitzen endständige funktionelle Gruppen, die zur Verknüpfung mit dem Protein und eine Vielzahl anderer funktioneller Gruppen, die zur Bindung der Farbstoffmoleküle genutzt werden. Als geeignete Rückgrat-Polymere werden Polyethylenimine, Polylysin und Polyamide wie Nylon 6 und niedermolekulare Polycarbonsäuren genannt.

Im ersten Beispiel dieser Anmeldung wird die Synthese eines Polymeren-Farbstoffes aus Polyethylenimin und Fluoresceinisothiocyanat beschrieben, der 70 Farbstoffmoleküle je Molekül Polyethylenimin enthält. Im Beispiel 7 wird jedoch die Quantenausbeute eines polymeren Farbstoffes mit 80 gebundenen Fluoresceineinheiten zu nur 4 % bestimmt. Daraus folgt, daß dieses Polymer bei etwa hundertfachem Molekulargewicht nur etwa dreimal so stark fluoresziert, wie monomeres FITC.

Im Gegensatz dazu erhält man erfindungsgemäß bei Bindung von 4-Aminofluorescein an Acrylamid-Maleinsäureanhydrid-Copolymere polymere Farbstoffe mit starker Fluoreszenz und Quantenausbeuten über 60 %.

Es wurden nun polymergebundene verknüpfte Farbstoffe gefunden bestehend aus
a) einem wasserlöslichen Polymer-Rückgrat,
b) daran covalent gebundenen Farbstoff sowie
c) funktionellen Gruppen, die eine covalente Verknüpfung der polymeren Farbstoffe mit biologischen Materialien ermöglichen,
worin das wasserlöslische Polymer Rückgrad ein Copolymer ist, welches als nicht-ionische Monomerbausteine Acrylamid, Methacrylamid, N-C₁-C₄-Alkyl-(meth)-acrylamide, N,N-C₁-C₄-Dialkylacrylamide, N-Vinylpyrrolidon, N-Vinylpiperidon, N-Vinylcaprolactam, N-Vinylformamid, N-Vinylacetamid, N-Vinyl-N-methyl-acetamid, N-Vinyl-O-methylurethan, Ethen oder Vinylmethylether und als reaktivgruppeneinführende Monomerbausteine, Maleinsäureanhydrid, Itaconsäureanhydrid, Citraconsäureanhydrid, (Meth)acrylsäurechlorid, (Meth)acrylsäure-N-hydroxysuccinimidester, (Meth)acrylsäure-N-hydroxyphthalimidester, N-(Meth)acryloylbenztriazol, 3- bzw. 4-Isothiocyanatophenyl(meth)acrylat, 2-Isocyanatoethylmethacrylat, Isocyanatostyrol, Isocyanatoisopropenyl-benzol, Vinyloxiran oder (Meth)acrylsäure in Kombination mit Carboddimiden enthält.

Die funktionellen Gruppen sind im allgemeinen über Spacer an das wasserlösliche Polymer-Rückgrat gebunden. Die polymeren Farbstoffe können zum Beispiel mit Proteinen oder funktionalisierten Oligonucleotiden verknüpft werden.

Dafür geeignete funktionelle Gruppen sind zum Beispiel Hydroxyl-, Amino-, Carboxyl- oder auch Thiogruppen sowie Isothiocyanatogruppen, ferner N-Hydroxysuccinimidester-, N-Hydroxyphthalimidester- bzw. N-Acylbenztriazolgruppen sowie falls eine Verknüpfung unter besonders schonenden, vorzugsweise wasserfreien Bedingungen möglich ist, auch Säurechlorid-, Säureanhydrid- und Isocyanatgruppen.

Die Polymeren bestehen aus covalent verknüpften (copolymerisierten) Monomerbausteinen, die dem Polymer die vorteilhaften Eigenschaften verleihen.
1. Wasserlöslich machende nicht-ionische Monomerbausteine wie Acrylamid, Methacrylamid, N-C₁-C₄-Alkyl(meth)acrylamide, N,N-C₁-C₄-Dialkylacrylamide, N-Vinylpyrrolidon, N-Vinylpiperidon, N-Vinylcaprolactam, N-Vinylformamid, N-Vinylacetamid, N-Vinyl-N-methyl-acetamid, N-Vinyl-O-methylurethan.
2. Über Ester- bzw. Säureimidgruppen oder vorzugsweise Säureamidgruppen covalent gebundende Farbstoffmoleküle.
   Die Farbstoffmoleküle können entweder durch Copolymerisation entsprechender Farbstoffmonomere wie (Meth)acrylsäureester oder (Meth)acrylamide geeigneter Farbstoffe oder durch Umsetzung von vorzugsweise Aminogruppen enthaltenden Farbstoffen mit Reaktivgruppen des Rückgratpolymers eingeführt werden.
   Reaktivgruppeneinführende Monomere sind Maleinsäureanhydrid, Itaconsäureanhydrid, Citraconsäureanhydrid, (Meth)acrylsäurechlorid, (Meth)acrylsäure-N-hydroxysuccinimidester, (Meth)acrylsäure-N-hydroxyphthalimidester, N-(Meth)acryloylbenztriazol, 3- bzw. 4-Isothiocyanatophenyl(meth)acrylat, 2-Isocyanatoethylmethacrylat, Isocyanatostyrol, Isocyanatoisopropenylbenzol, Vinyloxiran sowie (Meth)acrylsäure in Kombination mit Carbodiimiden.
3. Reaktive oder aktivierbare Gruppen enthaltende Monomerbausteine, die eine covalente Verknüpfung zum Beispiel mit den Antikörpern, Antigenen, Haptenen oder Nucleinsäuren sowie gegebenenfalls den Farbstoffmolekülen ermöglichen.
   Als solche können die obengenannten reaktivgruppeneinführenden Monomeren dienen, wenn sie nicht quantitativ mit Farbstoffmolekülen umgesetzt wurden und die Verknüpfung unter solchen Bedingungen durchgeführt werden kann, daß eine Hydrolyse der Reaktivgruppen zu vernachlässigen ist.
   Ferner können zu diesem Zweck vorzugsweise Monomere eingebaut werden, die eine aliphatische Alkoholgruppe enthalten, wie Hydroxialkyl(meth)acrylate wie Hydroxiethyl(meth)acrylat, Hydroxipropyl(meth)acrylat, Butandiolmono(meth)acrylat,
   Die Alkoholgruppe kann auch durch Umsetzung der unter 2. genannten Reaktivgruppen des Rückgratpolymeren mit Aminoalkoholen wie Aminoethanol, Aminopropanol oder 6-Amino-1-hexanol eingeführt werden.
   Die Alkoholgruppe des Polymeren kann durch Überführung in die Tresyl-(=Trifluormethansulfonyl-) oder Methansulfonylgruppe aktiviert werden.
4. Falls gewünscht, können ionische, die Wasserlöslichkeit des Polymeren erhöhende Monomerbausteine wie (Meth)acrylsäure, 2-Acryloylamino-2-methyl-propansulfonsäure, Styrolsulfonsäure, Dialkylaminoalkyl-(meth)acrylate bzw. Dialkylaminoalkyl-(meth)acrylamide wie Dimethylaminoethyl-methacrylat oder Dimethylaminopropylacrylamid bzw. die sich von diesen Monomeren ableitenden quaternierten (Meth)acrylate bzw. (Meth)acrylamide mit einpolymerisiert werden.

Zusätzliche Carboxyl-, Sulfonsäure- oder tert.-Aminogruppen können ferner durch Umsetzung eines Teils der Reaktivgruppen des Rückgratpolymers mit Aminocarbonsäuren, Aminosulfonsäuren bzw. primär-tertiären oder sekundär-tertiären Diaminen eingeführt werden.

Der Einbau ionischer Gruppen ist besonders bevorzugt, wenn die Polymere einen hohen Anteil gebundener hydrophober Farbstoffmoleküle enthalten.

Bevorzugte verknüpfbare anionische polymere Farbstoffe entsprechen der allgemeinen Formel 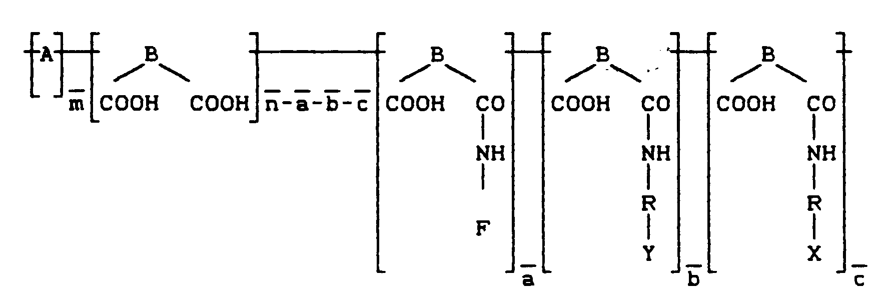
- A =: mit ungesättigten Dicarbonsäureanhydriden copolymerisierbare Monomere wie Acrylamid, Methacrylamid, N-C₁-C₄-Alkyl(meth)acrylamide, N,N-C₁-C₄-Dialkylacrylamide, N-Vinylpyrrolidon, N-Vinylpiperidon, N-Vinylcaprolactam, N-Vinylformamid, N-Vinylacetamid, N-Vinyl-N-methyl-acetamid, N-Vinyl-O-methylurethan sowie Ethen und Vinylmethylether, vorzugsweise Ethen, Methylvinylether, N-Vinylpyrrolidon, (Meth)acrylamid und N-Vinyl-N-methyl-acetamid.
besonders bevorzugt Acrylamid und Vinylpyrrolidon, da sie zu besonders gut wasserlöslichen Copolymeren führen;
- B =: Carboxylgruppenfreier Rest von Maleinsäure, Itaconsäure oder Citraconsäure,
besonders bevorzugt Maleinsäure;
- F =: Rest eines eine oder mehrere primäre und/oder sekundäre Aminogruppen enthaltenden Farbstoffes;
- Y =: gegenüber Antigenen bzw. endständig reaktiv funktionalisierten DNA (Gen-probes) reaktive Gruppe, besonders bevorzugt -O-SO₂-CH₃ und -O-SO₂-CH₂-CF₃;
- X =: die Wasserlöslichkeit des verknüpfbaren polymeren Farbstoffes erhöhende Gruppe, insbesondere -SO₃H-;
- R =: C₂-C₁₂-Alkylen, Cycloalkylen- oder Arylengruppe.

Eine weitere bevorzugte Gruppe verknüpfbarer polymere Farbstoffe entspricht der Formel 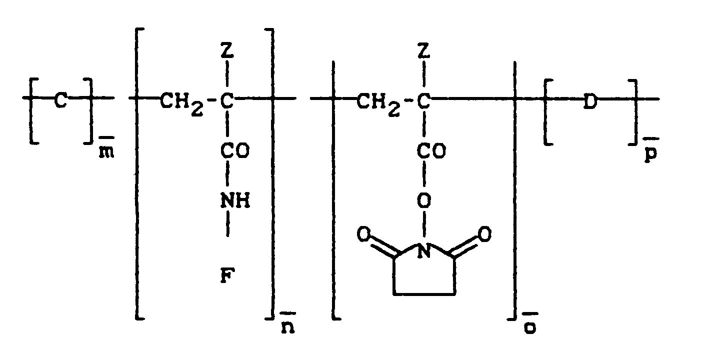
mit
- C =: Resten von (Meth)acrylamid, N-Vinylpyrrolidon, N-Vinyl-N-methylacetamid, besonders bevorzugt (Meth)acrylamid);
- Z =: H oder CH₃
- D =: (Meth)acrylsäure, 2-Acryloylamino-2-methylpropansulfonsäure, Styrolsulfonsäure, Dialkylaminoalkyl(meth)acrylate bzw. Dialkylaminoalkyl(meth)acrylamide bzw. die sich von diesen Monomeren ableitenden quaternierten (Meth)acrylate bzw. (Meth)acrylamide.

Das nachstehende Schema verdeutlicht ein Verfahren zur Herstellung der erfindungsgemäßen polymeren Farbstoffe.

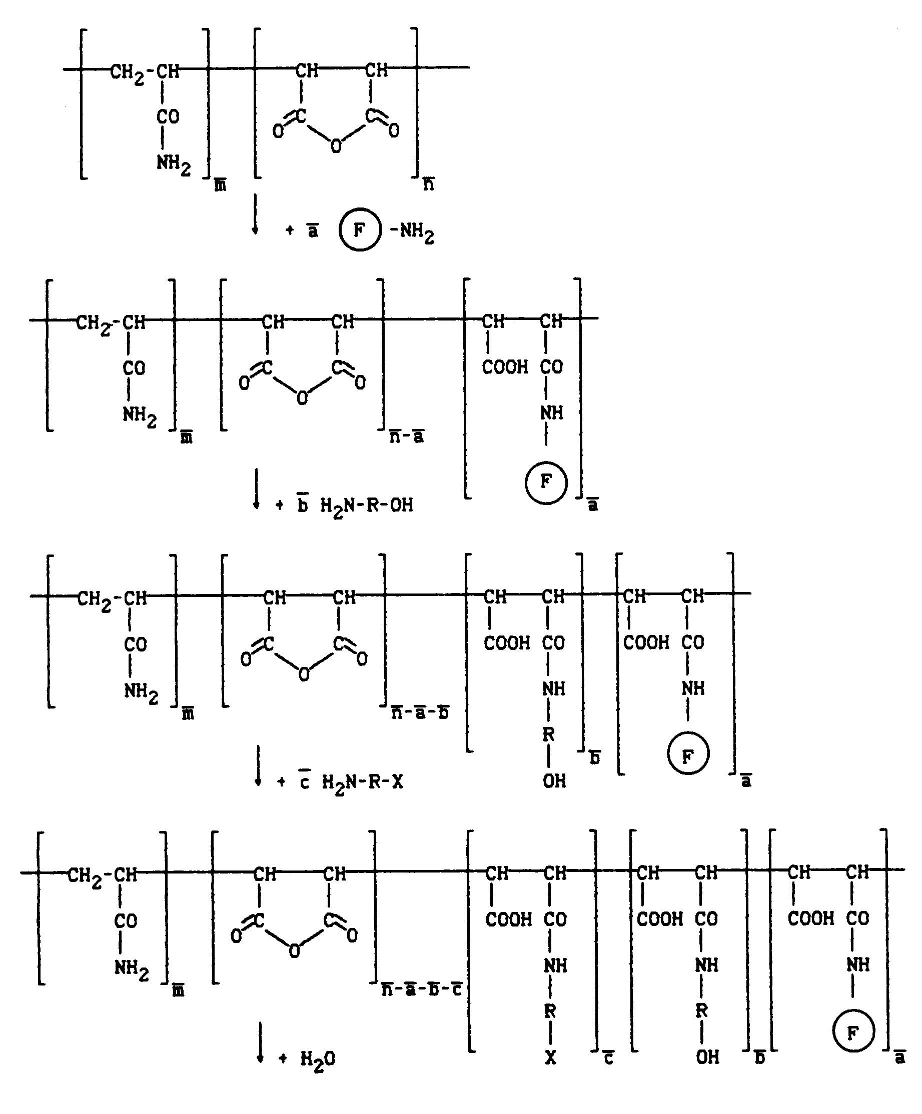

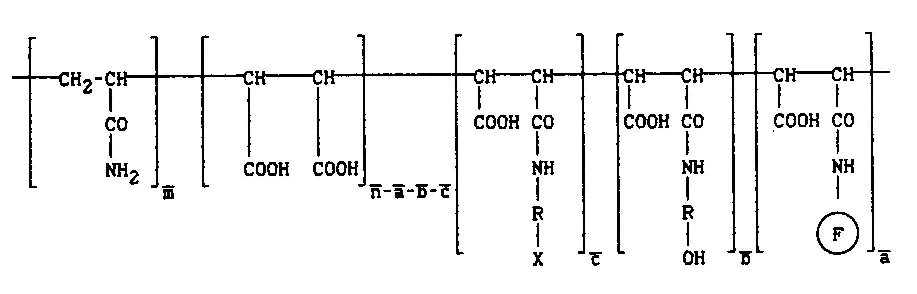

Statistische Copolymere aus Acrylamid und Maleinsäureanhydrid können durch radikalische Fällungspolymerisation in gegen Anhydridgruppen inerten Lösungsmitteln wie Aceton, Toluol oder Essigsäureethylester hergestellt werden. Zur Herstellung der erfindungsgemäßen polymeren Farbstoffe sind Copolymere mit m̅ = 25 bis 10⁴, vorzugsweise m̅ = 50 bis 10³ copolymerisierten Acrylamidmonomeren und n̅ = 3 bis 10³, vorzugsweise 5 bis 200 copolymerisierten Maleinsäureanhydridmonomeren geeignet. Das Verhältnis von m̅ : n̅ liegt üblicherweise zwischen 2:1 und 20:1.

Ⓕ steht stellvertretend für den Farbstoff und a̅ für die mittlere Anzahl von Farbstoffmolekülen pro Polymerkette. a̅ liegt zwischen 3 und 10³, vorzugsweise zwischen 5 und 500, besonders bevorzugt zwischen 10 and 200. a̅ ist immer um mindestens 1 kleiner als n̅, die Zahl der Maleinsäureanhydridmonomere pro Polymerkette.

Durch Umsetzung mit einem Aminoalkohol H₂N-R-OH werden Alkoholgruppen in den polymeren Farbstoff eingeführt. Die Zahl dieser Gruppen b̅ pro Polymerkette beträgt 1 bis 5, vorzugsweise 1 - 3.

Es ist möglich entweder die Umsetzung mit dem aminogruppenhaltigen Farbstoff oder mit dem Aminoalkohol zuerst durchzuführen.

Falls es erwünscht ist, können anschließend die noch verbliebenen Anhydridgruppen des Polymers mit einer Aminosulfonsäure (-R-X = -R-SO₃H), einer Aminocarbonsäure (-R-X =R-COOH) oder mit Ammoniak (-R-X = -H) umgesetzt werden. Die Anzahl c̅ dieser Moleküle kann 0 betragen. Vorzugsweise ist c̅ = 0 bis 100. Die Umsetzung mit Aminosulfonsäuren ist besonders bevorzugt, wenn die polymeren Farbstoffe eine große Anzahl hydrophober Farbstoffreste pro Polymerkette enthalten, da durch die eingeführten Sulfonsäuregruppen die Wasserlöslichkeit der polymeren Farbstoffe verbessert wird.

Die Umsetzungen des reaktiven Basispolymeren (z.B. des Acrylamid-Maleinsäureanhydrid-Copolymeren) mit dem Farbstoff (Ⓕ-NH₂), dem Aminoalkohol (H₂N-R-OH) und gegebenenfalls der Aminosäure oder Ammoniak können in homogener Lösung oder in Suspension durchgeführt werden. Das Lösungsmittel bzw. Suspensionsmedium sollte möglichst inert gegenüber den Reaktivgruppen des Basispolymeren sein um störende Nebenreaktionen zu vermeiden.

Bevorzugt sind solche Lösungsmittel bzw. Suspensionsmedien, die entweder eine Durchführung des gesamten Reaktionscyclus in homogener Lösung ermöglichen oder das Basispolymere bzw. den polymeren Farbstoff lösen. Nach Durchführung der Reaktionsfolge wird der polymere Farbstoff nach an sich bekannten Methoden isoliert durch Abdampfen des Lösungsmittels, vorzugsweise durch Ausfällen des polymeren Farbstoffes in einem geeigneten organischen Medium.

Das Verhältnis der gebundenen hydrophoben Farbstoffmoleküle a̅ zur Zahl der wasserlöslich machenden Polymerbestandteile m̅ = Acrylamid und c̅ = gebundene Aminosulfonsäure, Aminocarbonsäure oder Ammoniak wird so gewählt, daß die polymeren Farbstoffe die erforderliche Wasserlöslichkeit besitzen.

Die Abtrennung eines eventuellen Überschusses an Reagenzien kann entweder beim Ausfällen des polymeren Farbstoffs, durch erneutes Umfällen oder im Falle wasserlöslicher Reagenzien auch durch Dialyse oder Ultrafiltration erfolgen.

Anschließend wird der gereinigte polymere Farbstoff getrocknet.

Nach anschließender Überführung der Alkoholgruppen des polymeren Farbstoffes in Methansulfonyl-, Tresyl-, Trifluoracetyl-, Benzolsulfonyl- bzw. p-Toluolsulfonylgruppen, die nach bekannten Verfahren erfolgen kann, erhält man die erfindungsgemäßen verknüpfbaren polymeren Farbstoffe.

Analog zu den Acrylamid-Maleinsäureanhydrid-Copolymerisaten können auch weitere Dicarbonsäureanhydridgruppen enthaltende Copolymere aus Acrylamid, Methacrylamid, N-C₁-C₄-Alkyl(meth)acrylamiden, N,N-C₁-C₄-Dialkylacrylamiden, N-Vinylpyrrolidon, N-Vinylpiperidon, N-Vinylcaprolactam, N-Vinylformamid, N-Vinylacetamid, N-Vinyl-N-methyl-acetamid, N-Vinyl-O-methylurethan, Ethen oder Methylvinylether und Maleinsäureanhydrid, Itaconsäureanhydrid bzw. Citraconsäureanhydrid mit Aminogruppen enthaltenden Farbstoffen, Aminoalkoholen sowie gegebenenfalls Aminosulfonsäuren, Aminocarbonsäuren oder Ammoniak zu wasserlöslichen polymeren, verknüpfbaren Fabstoffen umgesetzt werden.

Auch Copolymere aus Acrylamid, Methacrylamid, N-C₁-C₄-Alkyl(meth)acrylamiden, N,N-C₁-C₄-Dialkylacrylamiden, N-Vinylpyrrolidon, N-Vinylpiperidon, N-Vinylcaprolactam, N-Vinylformamid, N-Vinylacetamid, N-Vinyl-N-methylacetamid oder N-Vinyl-O-methylurethan und (Meth)acrylsäurechlorid, Isocyanatoethylmethacrylat, Isocyanatostyrol, Isocyanatoisopropenylbenzol und Vinyloxiran können analog zu den Anhydridgruppen enthaltenden Copolymerisaten zu wasserlöslichen verknüpfbaren polymeren Farbstoffen umgesetzt werden. Dabei werden die Umsetzungen der Säurechloridgruppen enthaltenden Copolymere vorzugsweise in Gegenwart von tert.-Aminen als Säurefänger durchgeführt.

Copolymerisate aus Acrylamid, Methacrylamid, N-C₁-C₄-Alkyl(meth)acrylamiden, N,N-C₁-C₄-Dialkylacrylamiden, N-Vinylpyrrolidon, N-Vinylpiperidon, N-Vinylcaprolactam, N-Vinylformamid, N-Vinylacetamid, N-Vinyl-N-methyl-acetamid oder N-Vinyl-O-methylurethan und (Meth)acrylsäure-N-hydroxysuccinimidester, (Meth)acrylsäure-N-hydroxyphthalimidester, 1-N-(Meth)acryloylbenztriazol oder Isothiocyanatophenyl-(meth)acrylat können ebenfalls analog zu den Anhydridgruppen enthaltenden Copolymeren mit Aminogruppen enthaltenden Farbstoffen, Aminoalkoholen und gegebenenfalls Aminosulfonsäuren, Aminocarbonsäuren oder Ammoniak umgesetzt werden.

Bei diesen Copolymerisaten besteht jedoch zusätzlich die Möglichkeit, die Reaktivgruppen nur teilweise mit Aminogruppen enthaltenden Farbstoffen sowie gegebenenfalls Aminosulfonsäuren, Aminocarbonsäuren und/oder Ammoniak umzusetzen so daß 1 bis 5 Reaktivgruppen pro polymerem Farbstoffmolekül übrig bleiben. Die Verknüpfung mit den Antikörpern, Antigenen, Haptenen oder Nucleinsäuren erfolgen dann über diese Gruppen.

Außerdem können bei diesen Polymerisaten zusätzlich Hydroxialkyl(meth)acrylate einpolymerisiert werden. In diesem Falle können alle acylierenden Reaktivgruppen mit Farbstoffmolekülen sowie gegebenenfalls mit die Wasserlöslichkeit erhöhenden Verbindungen umgesetzt und die Alkoholgruppen anschließend in Methansulfonyl-, Tresyl-oder andere Reaktivgruppen überführt werden.

Eine weitere Möglichkeit zur Herstellung verknüpfbarer wasserlöslicher polymerer Farbstoffe besteht in der Copolymerisation von Farbstoffmonomeren, die außer dem Chromophor eine (Meth)acrylsäureester oder (Meth)acrylamid-Gruppe oder eine sonstige polymerisierbare Vinyl-bzw. Isopropenyl-Gruppe enthalten mit wasserlöslich machenden nicht-ionischen Monomerbausteinen wie Acrylamid, Methacrylamid, N-C₁-C₄-Alkyl(meth)acrylamiden, N,N-C₁-C₄-Dialkylacrylamiden, N-Vinylpyrrolidon, N-Vinylpiperidon, N-Vinylcaprolactam, N-Vinylformamid, N-Vinylacetamid, N-Vinyl-N-methyl-acetamid oder N-Vinyl-O-methylurethan, sowie mit einem weiteren Monomeren, welches eine gegen Wasser beständige reaktive oder eine aktivierbare Gruppe enthält. Die Monomerverhältnisse werden dabei so gewählt, daß im Mittel jede Polymerkette 1-5 reaktive bzw. aktivierbare Gruppen enthält und das Polymer wasserlöslich ist.

Die verwendbaren Farbstoffe, sind normalerweise solche, die im Ultravioletten, Infraroten oder sichtbaren Spektralbereich eine Absorption zeigen. Als Farbstoffe, die im sichtbaren Bereich absorbieren eignen sich insbesondere Azo-, Methin- sowie Anthrachinonfarbstoffe für den Einbau in die beschriebenen Polymeren. Sehr gut geeignet sind auch Heterozyklen enthaltende Methinfarbstoffe wie Oxazine, Thiazine, Triphendioxazine oder Chinophthalone. Diese Farbstoffe können sowohl wasserunlöslich sein als auch durch Sulfonsäure, Carbonsäure- bzw. kationische Gruppen Wasserlöslichkeit erhalten.

Bevorzugt sind Fluoreszens-Farbstoffe. Geeignete Farbstoffe sind zum Beispiel:
Cumarine der allgemeinen Formel 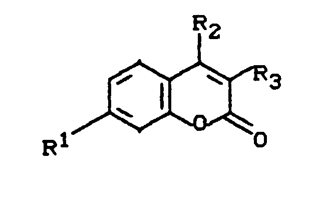
worin
R₁ für O-Alkyl, N(Alkyl)₂, NH-Alkyl, NH-SO₂-Alkyl, NH-SO₂-Aryl
R₂ für H, CN, Cl, OH, Alkylen, Aryl,
R₃ für Phenyl, Hetaryl steht.
R₁ kann auch noch 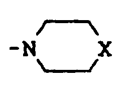
bedeuten, wobei
X für O, N-Alkyl oder
(CH₂)ₙ steht worin n 0 oder 1 sein kann.

Weiterhin gut geeignet sind Cumarine der Formel 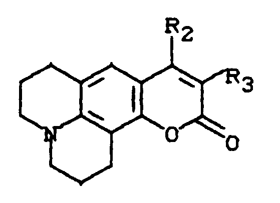
worin R₂ und R₃ die oben genannte Bedeutung haben.

Wenigstens einer der Substituenten R₁, R₂ oder R₃ soll eine funktionelle Gruppe für die Verknüpfung des Farbstoffs mit dem Polymer betragen. Die NH₂-Gruppe wird dafür als besonders geeignet angesehen.

Ebenfalls geeignet sind Carbostyrile der allgemeinen Formel 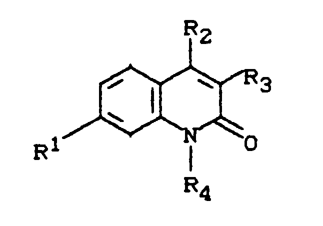
worin
- R₁, R₂ und R₃: die bei den Cumarinen angegebenen Bedeutung haben können und
- R₄: für Alkyl steht.

Auch hier muß einer der Substituenten ein eine funktionelle Gruppe für Verknüpfung mit dem Polymer tragen.

Weiterhin geeignet sind
Pyrazoline der allgemeinen Formel 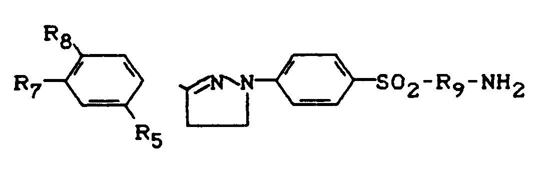
worin
R₅ = für H, oder CH₃,
R₇ und R₈ unabhängig voneinander für H oder Cl und
R₉ für Alkylen, 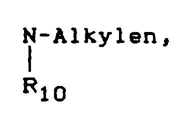
Alkylen-O-Alkylen steht
wobei
R₁₀ = Alkyl bedeutet.

Auch gut einsetzbar sind
Naphthalimide der allgemeinen Formel 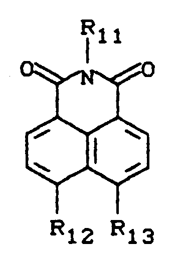
worin
R₁₁ für Alkyl,
R₁₂, R₁₃ für H, OAlkyl, N(Alkyl)₂ steht und
entweder die Alkylgruppe an R₁₁ oder jene an R₁₂ bzw. R₁₃ eine NH₂-Gruppe zur Verknüpfung mit dem Polymer trägt.

Als ebenfalls geeignet sind zu nennen
Pyrene der allgemeinen Formel 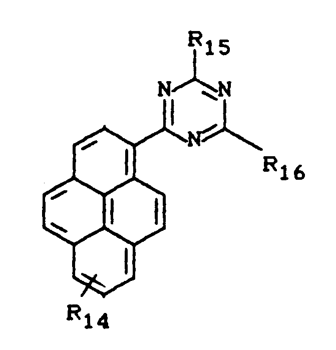
worin
- R₁₄: für H oder SO₃H,
- R₁₅ und R₁₆: unabhängig voneinander für OAlkyl oder N(Alkyl)₂ und
entweder eine Alkylgruppe an R₁₅ oder an R₁₆ eine NH₂-Gruppe zur Verknüpfung mit dem Polymer trägt.

Zu nennen wären noch Fluoresceine der Formel 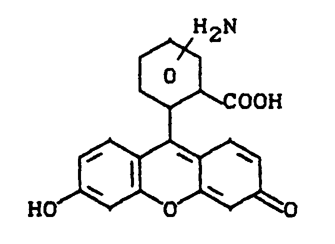
Auch geeignet sind Rhodamine der allgemeinen Formel 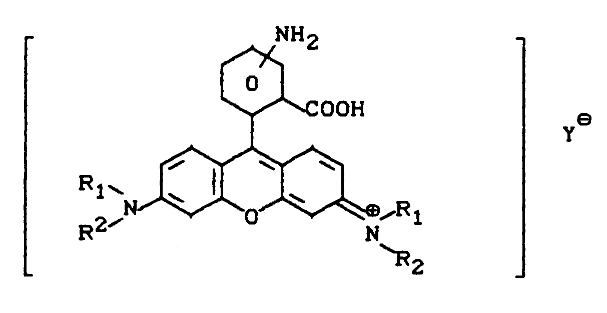
worin
- Y^{⊖}: ein farbloses Anion bedeutet und
- R₁ und R₂: für Alkyl, 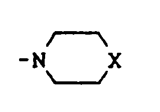 steht
wobei X für O, N-Alkyl oder (CH₂)ₙ mit n = 0 oder 1 steht.

R₁ und R₂ können auch zusammen mit den Aromaten einen Ring bilden wie zum Beispiel 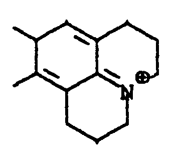
Diese und noch weitere Farbstoffe sind der Literatur wie z.B. "The Chemistry of Synthetic Dyes", Volume V, Akademie Press (1971), oder auch "Fluorescent Whitening Agents", vom Georg Thieme Verlag Stuttgart (1975).

Die erfindungsgemäßen Farbstoffe eignen sich zur Markierung in biologischen Analysenverfahren. So können z.B. Antikörper oder geeignet funktionalisierte Oligonucleotide farbig markiert werden, die dann in die üblichen Tests (Immunoassays bzw. Gensonden-Tests) eingesetzt werden können.

Ein für die Leistungsfähigkeit solcher Tests entscheidende Größe ist die Empfindlichkeit. Sie wird bei den meisten derzeit üblichen Testverfahren durch Einsatz radioaktiver Markierungssubstanzen erreicht.

Diese Methode hat in der praktischen Durchführung aber viele gravierende Nachteile (Strahlengefährdung, Zersetzlichkeit der Substanzen, schwierige Abfallentsorgung, Spezialausrüstung der Labors, spezielle Ausbildung des Personals), die eine Ausdehnung dieser vorteilhaften und leistungsfähigen Tests zu Routineverfahren bisher behindert haben. (s. z.B. WO 88-02784, Pharmacia, 21. 4. 88).

Es sind daher mehrere Versuche unternommen worden, die radioaktive Markierung durch eine problemlose Farbstoffmarkierung zu ersetzen. Damit umgeht man die Nachteile der radioaktiven Markierung, die erzielbare Empfindlichkeit ist aber für viele Tests nicht ausreichend [s. z.B. Nucleic Acids Res. 16, 4957 (1988)].

Hier bieten die erfindungsgemäßen polymeren Farbstoffe den Vorteil einer gesteigerten Empfindlichkeit ohne Verwendung von Radioaktivität.

Zur Verknüpfung der Polymeren Farbstoffe mit biologischen Substraten bieten sich mehrere Verfahren an.
1. Das Polymer enthält einpolymerisiert geeignete Gruppierungen, die zur Reaktion mit Antikörpern oder entsprechend funktionalisierten Oligonucleotiden befähigt sind, z.B. können Acrylester von N-Hydroxysuccinimid oder 1-N-Hydroxybenztriazol einpolymerisiert werden.
   Die aktiven Polymere werden nach Beladen mit Farbstoffen in wäßriger Lösung mit den zu markierenden biologischen Materialien umgesetzt. Die erhaltenen Mischungen können entweder direkt in Analysen verwendet werden oder nach vorheriger Reinigung.
2. Das Polymer enthält geeignete funktionelle Gruppen, die durch eine separate chemische Umsetzung so aktiviert werden, daß sie zur Verknüpfung mit Antikörpern und geeigent funktionalisierten Oligonucleotiden befähigt sind.
   Solche Gruppierungen können z.B. Hydroxygruppen sein, die sich durch Umsetzung nach literaturbekannten Verfahren in aktivierte Ester, z.B. Sulfonsäure- oder Carbonsäureester, überführen lassen.
   Dazu wird das Hydroxylgruppen-haltige Farbstoffpolymer in einem geeigneten Lösungsmittel, beispielsweise mit Methansulfonsäurechlorid, in das Mesylat umgewandelt.
   Dieses aktivierte Farbstoffpolymer wird dann in wäßriger Lösung mit dem biologischen Substrat (Antikörper bzw. geeignet funktionalisiertes Oligonucleotid) umgesetzt. Die erhaltene Mischung kann entweder direkt in Tests eingesetzt werden oder auch nach vorheriger Reinigung.

Geeignet funktionalisierte Oligonucleotide sind literaturbekannt. Darunter werden z.B. Oligonucleotide verstanden, die über einen inerten Spacer Amino-, Mercapto-oder Hydroxyfunktionen enthalten.

### Beispiel 1

### Copolymeres aus Acrylamid und Maleinsäureanhydrid:

57 g Acrylamid, 20 g Maleinsäureanhydrid und 0,77 g Azobisisobuttersäuredinitril werden in 500 ml trockenem Ethylacetat gelöst und die Lösung fitriert. Der Sauerstoff wird durch dreimaliges Evakuieren und Füllen mit Stickstoff entfernt und die Reaktionsmischung unter Stickstoff 20 Stunden bei 60° C gerührt. Das ausgefallene Copolymerpulver wird abgesaugt, gründlich mit Ethylacetat gewaschen und im Vakuum bei 50° C getrocknet.

Ausbeute: 56,3 g; N = 14,8 % =
25 Gew.-% Maleinsäureanhydrid,
Grenzviskosität [η] = 0,15 (gemessen in 0,9 %iger, wäßriger Kochsalzlösung) dies entspricht einem mittleren Molekulargewicht von etwa 20 000 Dalton.

### Beispiel 2

### Copolymeres aus N-Vinylpyrrolidon und Maleinsäureanhydrid

26,6 g N-Vinylpyrrolidon, 23,4 g Maleinsäureanhydrid und 116 g Methylenchlorid werden vorgelegt. Die Lösung wird wie in Beispiel A von Sauerstoff befreit und auf 40° C erwärmt. Innerhalb von 15 Minuten wird eine Lösung von 250 mg Dilauroyl-peroxid in 50 g Methylenchlorid zugetropft und dann 12 Stunden bei 40° C polymerisiert. Das ausgefallene Copolymerisat wird abfiltriert, mit Methylenchlorid gewaschen und im Vakuum bei 50° C getrocknet.

Ausbeute: 20,6 g, N = 6,8 %, entsprechend einem molaren Verhältnis von N-Vinylpyrrolidon zu Maleinsäureanhydrid von etwa 1:1; [η] = 0,11 (gemessen in DMF).

### Beispiel 3

### Copolymeres aus Acrylamid und Acryloyloxysuccinimid

54 g Acrylamid, 6 g Acryloyloxysuccinimid und 0,3 g Azobisisobuttersäurenitril werden in 400 ml trockenem Ethylacetat gelöst. Die Lösung wird wie in Beispiel A von Sauerstoff befreit und unter Stickstoff 20 Stunden bei 60° C gerührt. Das ausgefallene Copolymerpulver wird abgesaugt, gründlich mit Ethylacetat gewaschen und im Vakuum bei 50° C getrocknet.
Ausbeute: 58,4 g
Grenzviskosität [η] = 0,71 (gemessen in 0,8 %iger wäßriger Kochsalzlösung) dies entspricht einem mittleren Molekulargewicht von etwa 115 000 Dalton.

### Beispiel 4

1,5 g des Acrylamid-Maleinsäureanhydrid-Copolymers von Beispiel 1 werden in 30 ml trockenem Formamid bei 50° C gelöst. Nach dem Abkühlen werden bei Raumtemperatur 20 mg 6-Amino-1-hexanol zugegeben und der Ansatz 1 Stunde bei Raumtemperatur gerührt. Nach Zugabe von 600 mg 4-Aminofluorescein wird 24 Stunden bei Raumtemperatur und anschließend 5 Stunden bei 50° C weitergerührt. Der warme Ansatz wird in 1 l Aceton ausgefällt, das Polymer abgesaugt und in 40 ml Formamid bei 50° C gelöst. Nach Zugabe von 1 ml 25 %iger NH₄OH-Lösung wird 30 Minuten bei 50° C gerührt, der Ansatz abgekühlt und in 1 l Aceton erneut ausgefällt. Das Polymer wird abgesaugt, mit Aceton gewaschen und im Hochvakuum bei 25° C getrocknet. Die Fluoreszenz wird in Wasser bei pH 9 gemessen.
Ausbeute: 1,45 g; λₘₐₓ = 493 nm;
Extinktion bei 493 nm = 0,05 (4,5 mg polymerer Farbstoff in 1 l Wasser); Quantenausbeute = 0,63

1 g des so erhaltenen polymeren Fluoreszenzfarbstoffes werden bei Raumtemperatur in 50 ml trockenem Pyridin vorgelegt, mit 1 g Methansulfonsäurechlorid versetzt und unter Feuchtigkeitsausschluß 4 Stunden bei Raumtemperatur verrührt.

Anschließend wird abgesaugt und zweimal mit je 30 ml Isopropanol gewaschen.

Ausbeute: 0,9 g schwach gelbes, hygroskopisches Pulver,

Anstelle von Methansulfonsäurechlorid können zur Aktivierung des Farbstoffes nach analogem Verfahren auch Trifluormethansulfonsäurechlorid, Benzolsulfonsäurechlorid, Trifluoressigsäureanhydrid oder p-Toluolsulfonsäurechlorid verwendet werden.

### Beispiel 5

3 g des Acrylamid-Maleinsäureanhydrid-Copolymers von Beispiel 1 werden bei 50° C in 30 ml trockenem Formamid gelöst. Eine Suspension von 266 mg 3-(4-Aminophenyl)-7-Methylamino-cumarin in 2,5 ml trockenem DMF wird zugegeben und der Ansatz 3 Stunden bei 50° C gerührt. 30 mg 6-Amino-1-hexanol (fest) werden zugegeben und der Ansatz 1 Stunde bei 50° C weitergerührt. Die Lösung wird dann in 400 ml Ethylacetat eingerührt, wobei das Polymer ausfällt. Das Polymer wird abgesaugt, intensiv mit Ethylacetat gewaschen und im Vakuum über Phosphorpentoxid getrocknet.
Ausbeute: 2,8 g; λₘₐₓ = 389 nm;
Extinktion bei 389 nm = 0,05 (21 mg polymerer Farbstoff in 1 l Wasser); Quantenausbeute = 0,38.

1 g des so erhaltenen polymeren Fluoreszenzfarbstoffes werden in 100 ml trockenem Pyridin vorgelegt und mit 1 g Methansulfonsäurechlorid versetzt. Unter Feuchtigkeitsausschluß wird bei Raumtemperatur 4 Stunden gerührt, dann abgesaugt und mit je 30 ml Isopropanol 2 x gewaschen.
Ausbeute: 0,9 g farbloses, hygroskopisches Pulver.

### Beispiel 6

3 g des Acrylamid-Acryloyloxysuccinimid-Copolymers aus Beispiel 3 werden in 40 ml trockenem Formamid bei 50° C gelöst. Nach dem Abkühlen auf 25° C werden 0,3 g 4-Aminofluorescein zugegeben und der Ansatz 22 Stunden bei 25° C und 4 Stunden bei 50° C gerührt. Das Polymer wird in 500 ml Ethylacetat ausgefällt, nochmals in 30 ml Formamid gelöst und erneut in 500 ml Ethylacetat gefällt und nach der Isolierung im Vakuum über Phosphorpentoxid getrocknet.
Ausbeute: 2,4 g; λₘₐₓ = 493 nm;
Extinktion bei 493 nm = 0,05 (12 mg polymerer Farbstoff in 1 l Wasser bei pH 10); Quantenausbeute = 0,18
Mit ähnlichem Ergebnis läßt sich ein entsprechendes Acrylamid / 1-Acryloxy-benztriazol Copolymeres mit 4-Aminofluorescein markieren.

### Beispiel 7

2,09 g eines gemäß Beispiel 2 hergestellten Copolymerisats aus Maleinsäureanhydrid und N-Vinylpyrrolidon werden zusammen mit 1,356 g des Farbstoffes 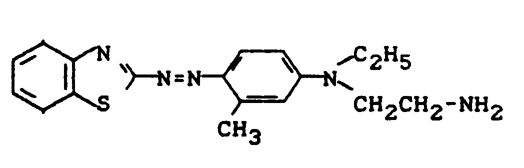
in 25 ml Formamid gelöst.

Nach 2-stündigem Rühren bei 50° C addiert man folgende Lösung zum Reaktionsgemisch:
52 mg 6-Amino-1-hexanol
500 mg 2-Aminoethansulfonsäure
5 ml Formamid
5 ml H₂O
Es wird noch 6 Stunden bei 50° C nachgerührt. Danach kühlt man auf Raumtemperatur ab und fällt den entstandenen polymeren Farbstoff durch Einrühren in einen 15fachen Überschuß an Aceton aus. Der abfiltrierte Farbstoff wird über Nacht im Exsikkator getrocknet.
Ausbeute: 3,56 g, λₘₐₓ = 510 nm
Extinktion bei 510 nm = 0.43 (40 mg polymerer Farbstoff in 1 l H₂O).

### Beispiel 8

1,5 g des Acrylamid-Maleinsäureanhydrid-Copolymers von Beispiel 1 werden in 30 ml trockenem Formamid bei 40-60° C gelöst.

Eine Lösung von 115 mg (3-(4-Aminophenyl)-7-diethylaminocumarin in 1,5 ml DMF wird zugegeben und die Lösung bei 50° C 1 Stunde gerührt. Nach Zugabe von 0,75 ml einer 1 %igen 3-Aminopropanollösung wird 30 Minuten weitergerührt und anschließend werden 0,5 ml konzentrierte wäßrige Ammoniaklösung zugegeben. Es wird 30 Minuten bei 50° C nachgerührt und der Ansatz in 750 ml Ethylacetat eingetropft. Der Polymerfarbstoff fällt feinteilig aus. Er wird abgesaugt, mit Ethylacetat gewaschen und rasch in einen Exiccator überführt, da er hydroskopisch ist. Die Trocknung erfolgt bei < 0,1 mbar.

Ausbeute: 1,42 g; λₘₐₓ = 412 nm;
Extinktion bei 412 nm = 0,07 (25 mg polymerer Farbstoff in 1 l H₂O); λ_{Emission} = 497 nm; Quantenausbeute = 0,21.

### Beispiel 9

1,5 g des Acrylamid-Maleinsäureanhydrid-Copolymers von Beispiel 1 werden in 30 ml trockenem Formamid bei 50° C gelöst. Nach Zugabe einer Lösung vo 115 mg 3-(4-Aminophenyl)-7-diethylaminocumarin in 1,5 ml DMF wird eine Stunde bei 50° C gerührt. 20 mg 6-Amino-1-hexanol (fest) werden zugegeben und der Ansatz 30 Minuten bei 50° C weitergerührt. Nach Zugabe von 300 mg Taurin (fest) wird weitere 60 Minuten bei 50° C gerührt und der Farbstoff anschließend wie in Beispiel 1 gefällt, isoliert und getrocknet.

Ausbeute: 1,85 g; λₘₐₓ = 414 nm;
Extinktion bei 414 nm = 0,07 (35 mg polymerer Farbstoff in 1l H₂O); λ_{Emission} = 501 nm; Quantenausbeute = 0,23 1 g des so hergestellten, Hydroxylgruppen enthaltenden polymeren Farbstoffes wird bei Raumtemperatur in 100 ml destilliertem trockenem Pyridin suspendiert. 1 ml Methansulfonsäurechlorid wird zugetropft und der Ansatz bei Raumtemperatur unter Lichtausschluß 4 Stunden gerührt. Anschließend wird rasch abgesaugt, zweimal mit je 30 ml Isopropanol gewaschen und im Ölpumpenvakuum getrocknet.

Ausbeute: 0,95 g schwach gelbes, hygroskopisches Pulver.

### Beispiel 10

1,5 g des Acrylamid-Maleinsäureanhydrid-Copolymers von Beispiel 1 werden in 35 ml trockenem Formamid bei 60° C gelöst. Eine Lösung von 300 mg 4-Aminofluorescein in 3 ml DMF wird zugegeben und der Ansatz 1 Stunde bei 60° C gerührt. Nach Zugabe von 15 mg 6-Amino-1-hexanol (fest) wird 30 Minuten bei 60° C weiter gerührt. 300 mg Taurin (fest) werden zugegeben und 30 Minuten bei 60° C nachgerührt. Anschließend wird der polymere Farbstoff wie in Beispiel 1 beschrieben isoliert und getrocknet.

Ausbeute: 1,75 g; λₘₐₓ = 476 nm;
Extinktion bei 476 nm = 0,04 (50 mg polymerer Farbstoff in 1l H₂O); λ_{Emission} = 518 nm;
Quantenausbeute = 0,22.

### Beispiel 11

2,09 g des Copolymerisats aus N-Vinylpyrrolidon und Maleinssäureanhydrid von Beispiel 2 werden zusammen mit 131 mg des Farbstoffs 3-(4-Aminophenyl)-7-diethylaminocumarin in 6,66 g Formamid bei 50° C umgesetzt.

Nach 1-stündigem Rühren bei 50° C gibt man folgende Lösung zum Reaktionsgemisch:
19 mg 6-Amino-1-hexanol (fest)
960 mg einer wäßrige Lösung von Na-Taurin (Festgehalt: 43 %)
3,0 g Formamid
3,0 g Wasser.

Es wird noch 5 Stunden bei 50° C nachgerührt. Dann kühlt man auf Raumtemperatur ab und fällt den polymeren Farbstoff durch Einrühren in einen Überschuß von Aceton aus. Das Produkt wird im Exsikkator über Nacht getrocknet.
Ausbeute: 2,40 g, λₘₐₓ = 411 nm;
Extinktion bei 411 nm = 0,05 (20 mg polymerer Farbstoff in 1 l H₂O); λ_{Emission} = 500 nm;
Quantenausbeute = 0,30.

### Beispiel 12

5 g des Acrylamid-Maleinsäureanhydrid-Copolymers von Beispiel 1, 150 ml wasserfreies Formamid und 500 mg des Farbstoffes 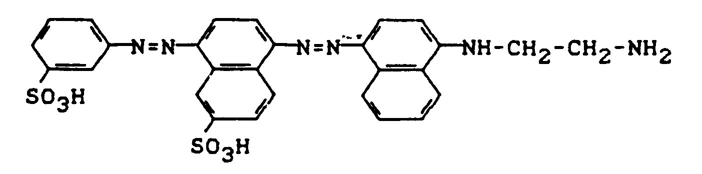
werden unter Stickstoff 2 Stunden bei 120° C gerührt. Nach dem Abkühlen auf 60° C werden 40 mg 6-Amino-1-hexanol (fest) zugegeben und eine Stunde bei 60° C weitergerührt. 2 ml konzentrierte wäßrige Ammoniaklösung werden zugegeben und nach einer weiteren Stunde bei 60° C wird der polymere Farbstoff in 1,5 l Ethylacetat ausgefällt und wie in Beispiel 1 isoliert und getrocknet.

Ausbeute: 5,5 g; λₘₐₓ = 568 nm;
Extinktion bei 568 nm = 0,67 (0,2 mg polymerer Farbstoff in 1 ml Wasser).

### Beispiel 13

1,25 g des Acrylamid-Maleinsäureanhydrid-Copolymers von Beispiel 1 werden in 50 ml trockenem Formamid bei 60° C gelöst. 10 mg 6-Amino-1-hexanol (fest) werden zugegeben. Nach 1 Stunde bei 60° C werden 0,75 g des Farbstoffes 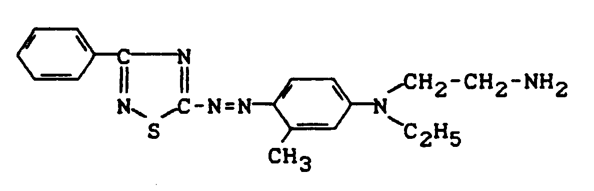
zugegeben und der Ansatz 4 Stunden bei 60° C weitergerührt. Dann werden 500 mg Taurin (fest) zugegeben und 1 Stunde bei 60° C nachgerührt. Der polymere Farbstoff wird durch Eintropfen der Reaktionslösung in 1,5 l Aceton ausgefällt und wie in Beispiel 1 beschrieben isoliert und getrocknet.

Ausbeute: 1,75 g; λₘₐₓ = 533 nm;
Extinktion bei 533 nm = 0,7 (0,2 mg polymerer Farbstoff in 1 ml Wasser).

### Beispiel 14

1,26 g eines alternierenden Copolymerisates aus Maleinsäureanhydrid und Ethylen mit dem Molekulargewicht 25.000 (EMA®21 der Firma Monsanto) werden in 20 ml Formamid gelöst. Zu dieser Lösung gibt man eine Lösung von 1,695 g eines Farbstoffes mit der Formel 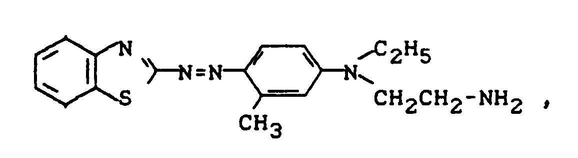
ebenfalls gelöst in 20 ml Formamid. Das Reaktionsgemisch wird 1 Stunde bei Raumtemperatur und 2 Stunden bei 50° C gerührt. Danach addiert man 117,6 mg 6-Amino-1-hexanol, gelöst in 5 ml Formamid, und rührt weitere 30 Minuten. Schließlich gibt man 500 mg 2-Aminoethansulfonsäure und nach einer weiteren halben Stunde 5 ml H₂O zum Reaktionsgemisch und rührt noch 6 Stunden bei 50° C nach. Nach Abkühlen auf Raumtemperatur wird der gebildete polymere Farbstoff durch Einrühren in ca. 1,5 l Aceton ausgefällt. Nach dem Abfiltrieren wird der Farbstoff bis zur Gew.-Konstanz im Vakuumexsikkator getrocknet.
Ausbeute: 3,21 g; λₘₐₓ = 495 nm;
Extinktion bei λₘₐₓ = 1,08 (Konzentr.: 40 mg/l; LM : H₂O). Molarer Extinktionskoeffizient: 1,9 · 10⁶.

1 g des so hergestellten polymeren Farbstoffes wird bei Raumtemperatur in 50 ml trockenem Pyridin mit 1 g Methansulfonsäurechlorid 4 Stunden unter Feuchtigkeitsausschluß verrührt. Anschließend wird abgesaugt, zweimal mit 30 ml Isopropanol gewaschen und im Ölpumpenvakuum bei Raumtemperatur getrocknet.
Ausbeute: 0,9 g schwarzes, hygroskopisches Pulver.

### Beispiel 15

1,56 g eines alternierenden Copolymerisates aus Maleinsäureanhydrid und Methylvinylether mit dem Molekulargewicht 20.000 (Gantrez®AN-119 der Firma GAF) werden in 20 ml Formamid gelöst. Zu dieser Lösung werden 2,034 g einer Lösung des in Beispiel 14 verwendeten Farbstoffes in 30 ml Formamid gegeben. Nach einstündigem Rühren bei Raumtemperatur und zweistündigem Rühren bei 50° C wird folgendes Reaktionsgemisch zugegeben:
235 mg 6-Amino-1-hexanol
250 mg 2-Aminoethansulfonsäure
5 ml Formamid
5 ml H₂O.

Nach sechsstündigem Rühren bei 50° C wird die Lösung auf Raumtemperatur abgekühlt und in ca. 1,2 l Ethylacetat eingerührt. Dabei fällt der polymere Farbstoff als feines Pulver aus, das abfiltriert und im Vakuum bei Raumtemperatur bis zur Gew.-Konstanz getrocknet wird.
Ausbeute: 1,25 g; λₘₐₓ = 493 nm;
Extinktion bei λₘₐₓ = 0,788 (Konzentr.: 40 mg/l; LM : H₂O). Molarer Extinktionskoeffizient: 0,97 · 10⁶.

1 g des so hergestellten polymeren Farbstoffes wird bei Raumtemperatur in 70 ml trockenem Pyridin mit 2 g Methansulfonsäurechlorid 4 Stunden unter Feuchtigkeitsausschluß verrührt. Anschließend wird abgesaugt, zweimal mit 30 ml Isopropanol gewaschen und im Ölpumpenvakuum bei Raumtemperatur getrocknet.
Ausbeute: 0,9 g schwarzes, hygroskopisches Pulver.

### Beispiel 16

0,5 mg monoklonale Antikörper gegen CEA (Carcinogen Embryonal Antigen) werden in 1 ml H₂O mit 0,86 mg aktiviertem Farbstoff gemäß Beispiel 4 1 Stunde bei Raumtemperatur inkubiert, gegen Wasser dialysiert und lyophilisiert. Der so erhaltene markierte monoklonale Antikörper wurde durch UV-Spektroskopie charakterisiert.

Im immunologischen Test auf CEA erzielt man bei Verwendung dieses markierten Antikörpers eine erhöhte Empfindlichkeit verglichen mit analogen monomeren Farbstoffen.

Mit ähnlichen Resultaten läßt sich der CEA monoklonale Antikörper auch mit Farbstoffen gemäß den Beispielen 5, 6, 7, 9, 14 und 15 markieren.

### Beispiel 17

Es werden 500 µg eines Aminolink-Oligonucleotids, abgeleitet von der Sequenz: GCCGCCTCGG CCTCGCCGAC GCCCGGGACG GGCGCCACCC CCAACGACGT (geeignet als Gensonden-Test für Pseudorabies-Vinus) [Synthese: E.Sonveaux, Bioorganic Chemistry 14, 274 (1986) sowie N. D. Sinha u. R. M. Cook, Nucleic Acids Research 16, 2659 (1988) in 200 µl Carbonat-Puffer (pH = 9) gelöst. Dazu gibt man einen Überschuß eine Lösung des polymeren Fluoreszenz-Farbstoffes gemäß Beispiel 6 in 300 µl Formamid und rührt 36 Stunden bei Raumtemperatur. Die Aufarbeitung erfolgt durch Gelfiltration on BIORAD-Bio-Gel P 4 und anschließende RP-HPLC Reinigung.

Bei Verwendung des so markierten Aminolink-Oligonucleotids beim DNA-Probe-Test auf Pseudorabies-Virus wird eine verbesserte Sensitivität erreicht als bei Einsatz von mit entsprechenden monomeren Fluroeszenz-Farbstoffen markierter DNA.

Mit ähnlichen Resultaten läßt sich das Aminolinknucleotid auch mit aktivierten polymeren Farbstoffen gemäß den Beispielen 4, 5, 7, 9, 14 und 15 markieren und bei DNA-Probe-Tests einsetzen.

## Patentansprüche

1. Polymer gebundene verknüpfbare Farbstoffe bestehend aus
a) einem wasserlöslichen Polymer-Rückgrat,
b) daran covalent gebundenen Farbstoffen sowie
c) funktionellen Gruppen, die eine covalente Verknüpfung der polymeren Farbstoffe mit biologischen Materialien ermöglichen,
worin das wasserlösliche Polymer Rückgrat ein Copolymer ist welches als nicht-ionische Monomerbausteine Acrylamid, Methacrylamid, N-C₁-C₄-Alkyl-(meth)-acrylamide, N,N-C₁-C₄-Dialkylacrylamide, N-Vinylpyrrolidon N-Vinylpiperidon N-Vinylcaprolactam, N-Vinylformamid,, N-Vinylacetamid, N-Vinyl-N-methyl-acetamid, N-Vinyl-O-methylurethan, Ethen oder Vinylmethylether und als reaktivgruppeneinführende Monomerbausteine Maleinsäureanhydrid, Itaconsäureanhydrid, Citraconsäureanhydrid, (Meth)acrylsäurechlorid, (Meth)acrylsäure-N-hydroxysuccinimidester, (Meth)acrylsäure-N-hydroxy-phthalimidester, N-(Meth)acryloylbenztriazol, 3-bzw. 4-Isothiocyanatophenyl(meth)acrylat, 2-Isocyanatoethylmethacrylat, Isocyanatostyrol, Isocyanatoisopropenyl-benzol, Vinyloxiran oder (Meth)acrylsäure in Kombination mit Carbodiimiden enthält.

2. Polymer gebundene verknüpfbare Fluoreszenzfarbstoffe gemäß Anspruch 1 enthaltend einen Farbstoff aus der Gruppe der Cumarine, Carbostyrile, Pyrazoline, Naphthalimide, Pyrene, Fluoresceine oder Rhodanine.

3. Polymer gebundene verknüpfbare Farbstoffe gemäß Anspruch 1 enthaltend einen Farbstoff aus der Gruppe der Azo-, Methin- oder Anthrachinonfarbstoffe.

4. Polymer gebundene verknüpfbare Farbstoffe gemäß Anspruch 1 enthaltend funktionelle Gruppen aus der Gruppe der Hydroxyl-, Amino-, Carboxyl-, Thio- und Isothiocyanatgruppen.

5. Polymer gebundene verknüpfbare Farbstoffe gemäß Anspruch 1 wobei
a) das wasserlösliche Polymerrückgrat ein Acrylamid-Maleinsäureanhydrid-Copolymer ist
b) der Farbstoff aus der Gruppe Coumarine, Fluoresceine und Rhodamine stammt und
c) die funktionellen Gruppen durch Umsetzung mit Aminoalkoholen eingeführt werden.

6. Polymer gebundene verknüpfbare Farbsttoffe gemäß Anspruchen 1 wobei
a) das wasserlösliche Polymerrückgrat ein Vinylpyrolidon-Maleinsäureanhydrid-Copolymer ist
b) der Farbstoff aus der Gruppe Coumarine, Fluoresceine und Rhodamine stammt und
c) die funktionellen Gruppen durch Umsetzung mit Aminoalkoholen eingeführt werden.

7. Antikörper verknüpft mit einem Polymer gebundenen Farbstoff gemäß einem der Ansprüche 1 bis 6.

8. Nucleinsäure verknüpft mit einem Polymer gebundenen Farbstoff gemäß einem der Ansprüche 1 bis 6.

## Claims

1. Polymer-bound linkable dyes comprising
a) a water-soluble polymer backbone,
b) dyes covalently bonded thereto and
c) functional groups which make possible covalent linking of the polymeric dyes to biological materials,
in which the water-soluble polymer backbone is a copolymer containing as non-ionic monomer units acrylamide, methacrylamide, N-C₁-C₄-alkyl(meth)-acrylamides, N,N-C₁-C₄-dialkylacrylamides, N-vinylpyrrolidone, N-vinylpiperidone, N-vinylcaprolactam, N-vinylformamide, N-vinylacetamide, N-vinyl-N-methylacetamide, N-vinyl-O-methylurethane, ethene or vinyl methyl ether and as reactive group introducer monomer units maleic anhydride, itaconic anhydride, citraconic anhydride, (meth)acryloyl chloride, N-hydroxysuccinimidyl (meth)acrylate, N-hydroxyphthalimidyl (meth)acrylate, N-(meth)acryloylbenzotriazole, 3- or 4-isothiocyanatophenyl (meth)acrylate, 2-isocyanatoethyl methacrylate, isocyanatostyrene, isocyanatoisopropenylbenzene, vinyloxirane or (meth)acrylic acid in combination with carbodiimides.

2. Polymer-bound linkable fluorescent dyes according to Claim 1 containing a dye from the group of the coumarins, carbostyrils, pyrazolines, naphthalimides, pyrenes, fluoresceins or rhodanines.

3. Polymer-bound linkable dyes according to Claim 1 containing a dye from the group of the azo dyes, methine dyes or anthraquinone dyes.

4. Polymer-bound linkable dyes according to Claim 1 containing functional groups from the group of the hydroxyl, amino, carboxyl, thio and isothiocyanate groups.

5. Polymer-bound linkable dyes according to Claim 1 in which
a) the water-soluble polymer backbone is an acrylamide-maleic anhydride copolymer
b) the dye comes from the group consisting of coumarins, fluoresceins and rhodamines and
c) the functional groups are introduced by reaction with amino alcohols.

6. Polymer-bound linkable dyes according to Claim 1 in which
a) the water-soluble polymer backbone is a vinyl pyrrolidone-maleic anhydride copolymer
b) the dye comes from the group consisting of coumarins, fluoresceins and rhodamines and
c) the functional groups are introduced by reaction with amino alcohols.

7. Antibody linked to a polymer-bound dye according to any one of Claims 1 to 6.

8. Nucleic acid linked to a polymer-bound dye according to any one of Claims 1 to 6.

## Revendications

1. Colorants fixés à des polymères et aptes à des liaisons, qui consistent en :
a) un polymère de squelette soluble dans l'eau,
b) des colorants fixés sur ce polymère par des liaisons covalentes, et
c) des groupes fonctionnels permettant une liaison covalente entre les colorants polymères et des substances biologiques,
le polymère de squelette soluble dans l'eau étant un copolymère contenant en tant que motifs de structure monomères non ioniques l'acrylamide, le méthacrylamide, un N-(alkyle en C₁-C₄)-(méth)acrylamide, un N,N-di-(alkyle en C₁-C₄)-acrylamide, la N-vinylpyrrolidone, la N-vinylpipéridone, le N-vinylcaprolactame, le N-vinylformamide, le N-vinylacétamide, le N-vinyl-N-méthylacétamide, le N-vinyl-O-méthyluréthanne, l'éthylène ou l'éther vinyl-méthylique et en tant que motifs de structure monomères introduisant des groupes réactifs l'anhydride maléique, l'anhydride itaconique, l'anhydride citraconique, le chlorure de l'acide (méth)acrylique, l'ester de N-hydroxysuccinimide de l'acide (méth)acrylique, l'ester de N-hydroxyphtalimide de l'acide (méth)acrylique, le N-(méth)acryloylbenzotriazole, le (méth)acrylate de 3- ou 4-isothiocyanatophényle, le méthacrylate de 2-isocyanatoéthyle, l'isocyanatostyrène, l'isocyanatoisopropénylbenzène, le vinyloxiranne ou l'acide (méth)acrylique en combinaison avec des carbodiimides.

2. Colorants fluorescents fixés sur des polymères et aptes à des liaisons selon la revendication 1, contenant un colorant du groupe des coumarines, des carbostyryles, des pyrazolines, des naphtalimides, des pyrènes, des fluorescéines ou des rhodamines.

3. Colorants fixés sur des polymères et aptes à des liaisons selon la revendication 1, contenant un colorant du groupe des colorants azoïques, des colorants de méthines ou des colorants d'anthraquinones.

4. Colorants fixés sur des polymères et aptes à des liaisons selon la revendication 1, contenant des groupes fonctionnels choisis parmi les groupes hydroxy, amino, carboxyle, thio- et isothiocyanato.

5. Colorants fixés sur des polymères selon la revendication 1 pour lesquels :
a) le polymère de squelette soluble dans l'eau est un copolymère acrylamide/anhydride maléique,
b) le colorant est pris dans le groupe des coumarines, des fluorescéines et des rhodamines, et
c) les groupes fonctionnels sont introduits par réaction avec des aminoalcools.

6. Colorants fixés sur des polymères et aptes à des liaisons selon la revendication 1, pour lesquels :
a) le polymère de squelette soluble dans l'eau est un copolymère vinylpyrrolidone/anhydride maléique,
b) le colorant est pris dans le groupe des coumarines, des fluorescéines et des rhodamines, et
c) les groupes fonctionnels sont introduits par réaction avec des aminoalcools.

7. Anticorps reliés à un colorant fixé sur un polymère selon une des revendications 1 à 6.

8. Acide nucléique relié à un colorant fixé sur un polymère selon une des revendications 1 à 6.
